(19) Europäisches Patentamt
European Patent Office
Office européen des brevets

(11) EP 4 276 447 A1

(12) **EUROPEAN PATENT APPLICATION**

(43) Date of publication:
**15.11.2023 Bulletin 2023/46**

(21) Application number: **23172753.8**

(22) Date of filing: **11.05.2023**

(51) International Patent Classification (IPC):
***G01N 21/64*** *(2006.01)* ***G01N 33/543*** *(2006.01)*
***G01N 33/551*** *(2006.01)*

(52) Cooperative Patent Classification (CPC):
**G01N 21/6445; G01N 33/543; G01N 33/551**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.05.2022 JP 2022079605**
**14.04.2023 JP 2023066556**

(71) Applicant: **CANON KABUSHIKI KAISHA**
**Tokyo 146-8501 (JP)**

(72) Inventors:
- **KAKEGAWA, Norishige**
**Tokyo 146-8501 (JP)**
- **MASUMURA, Takahiro**
**Tokyo 146-8501 (JP)**
- **YAMAUCHI, Fumio**
**Tokyo 146-8501 (JP)**
- **KANAZAKI, Kengo**
**Tokyo 146-8501 (JP)**
- **NAKAMURA, Tomohiro**
**Tokyo 146-8501 (JP)**
- **NAKAJIMA, Ikuo**
**Tokyo 146-8501 (JP)**
- **SAKAKIBARA, Teigo**
**Tokyo 146-8501 (JP)**

(74) Representative: **TBK**
**Bavariaring 4-6**
**80336 München (DE)**

## (54) ANALYSIS METHOD INVOLVING MEASUREMENT BASED ON POLARIZATION ANISOTROPY, TEST KIT, AND TEST REAGENT

(57) Provided are a reagent and a measurement method for enabling a specimen test based on polarization anisotropy to be performed with high sensitivity and within a short period of time. Specifically, provided is an analysis method including measuring a value (R) for polarization anisotropy through use of a luminescent reagent that binds with a target substance, the analysis method including: a reaction step including mixing a sample containing the target substance with the luminescent reagent and a sensitizer, and subjecting the mixture to a reaction to obtain a reaction liquid; and a measurement step of measuring the R of the reaction liquid, the luminescent reagent including a luminescent particle substrate and a hydrophilic layer arranged on an outside of the luminescent particle substrate, the sensitizer containing a hydrophilic polymer.

FIG. 1

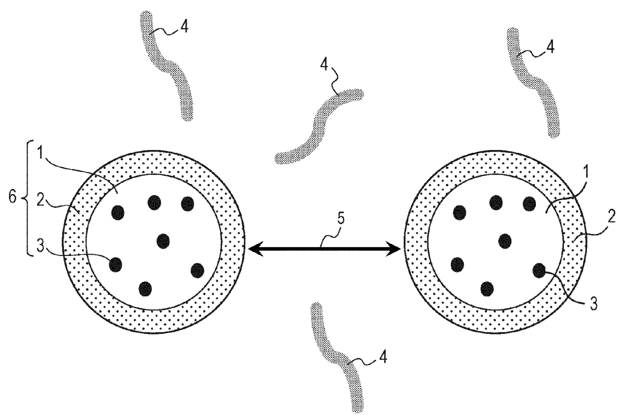




Processed by Luminess, 75001 PARIS (FR)

## Description

BACKGROUND OF THE INVENTION

Field of the Invention

The present invention relates to an analysis method involving measurement based on polarization anisotropy, a test kit, and a test reagent.

Description of the Related Art

In the fields of medicine and clinical tests, high-sensitivity detection or quantification of a trace amount of a biological component from, for example, blood or a collected part of an organ is required for investigating, for example, the cause and presence or absence of a disease.

Among test techniques for biological components, immunoassays are widely utilized. In many of the immunoassays, a washing step called bound/free (B/F) separation is required. As an immunoassay that does not require the B/F separation, there is known a latex agglutination method utilizing an antigen-antibody reaction. In the latex agglutination method, latex particles each having supported thereon, for example, an antibody that specifically binds to a target substance are mixed with a liquid that may contain the target substance, and the degree of agglutination of the latex particles is measured.

In the latex agglutination method, the target substance is captured by the antibody bound to the latex particles and specific to the target substance, and a plurality of the latex particles are crosslinked via the captured target substance, with the result that the agglutination of the latex particles occurs. That is, the amount of the target substance in a liquid sample such as a biological sample can be quantified by evaluating the degree of the agglutination of the latex particles. The degree of the agglutination can be quantified by measuring and evaluating a change in amount of light transmitted through or scattered by the liquid sample.

The latex agglutination method can detect/quantitatively evaluate an antigen as the target substance in a simple and rapid manner, but has involved a problem with detection limits in that the antigen cannot be detected when its amount in the liquid sample such as the biological sample is small.

In order to improve detection sensitivity for the target substance, it is required that the degree of the agglutination be measured with higher sensitivity. That is, it is conceivable to replace a system for measuring the change in amount of the light transmitted through or scattered by the liquid sample with a method for detection/quantification utilizing a luminescence characteristic with higher sensitivity. Specifically, there has been proposed a specimen test method utilizing a fluorescence depolarization measurement (Japanese Patent Publication No. H03-52575 and Japanese Patent No. 2893772).

In Japanese Patent Publication No. H03-52575, it is proposed that an apparatus for the fluorescence depolarization measurement be improved to be clinically used.

In the fluorescence depolarization measurement, the B/F separation required in a general fluorescence measurement method is not required.

Accordingly, use of the fluorescence depolarization measurement enables a simple specimen test as with the latex agglutination method. Further, it is conceived that use of the fluorescence depolarization measurement enables measurement by the same test system as in the latex agglutination method by merely mixing a luminescent substance that specifically reacts with the target substance in a measurement process. Meanwhile, in Japanese Patent Publication No. H03-52575, there is a proposal of use of a single molecule such as fluorescein as a luminescent material, which is applicable only to a drug, a low-molecular-weight antigen, and the like in principle.

Japanese Patent No. 2893772 has solved the problem of Japanese Patent Publication No. H03-52575, i.e., the problem in that the fluorescence depolarization measurement is applied only to a drug, a low-molecular-weight antigen, and the like. That is, in Japanese Patent No. 2893772, with an aim to apply the fluorescence depolarization measurement to a macromolecule such as a protein, it is proposed to use, as a luminescent material, a material obtained by adsorbing a dye having a long-lifetime luminescence characteristic onto latex particles. In Japanese Patent No. 2893772, it is proposed that a high-molecular-weight substance be quantified by balancing a reduction in rotational Brownian motion of the substance in a liquid due to an increase in particle diameter and the length of emission lifetime based on the principle of the fluorescence depolarization measurement. However, in Japanese Patent No. 2893772, a fluorescent substance is supported on the latex particles after synthesis of the particles, and hence an interaction between fluorescent substances adsorbed in the vicinity of surfaces of the particles or the like makes it difficult to stably determine the polarization anisotropic property of the testing particles. Further, in Japanese Patent No. 2893772, bovine serum albumin (BSA), which is a biomolecule, is supported on surfaces of the particles in order to suppress nonspecific adsorption, and hence there is a risk in that a lot-to-lot variation may occur owing to a broad particle size distribution and

BSA, which is a protein.

Accordingly, measurement is performed with the concentration of the target substance being on the order of μg/mL, which is not greatly different from the latex method in terms of measurement sensitivity.

In addition, when high sensitivity measurement is performed by the fluorescence depolarization measurement, the amount of a fluorescent reagent to be reacted needs to be adjusted in accordance with the amount of the target substance. This is because, when a large amount of an unreacted fluorescent reagent is contained after reaction with the target substance, a change in value for fluorescence anisotropy is observed to be small as a whole. Meanwhile, the binding rate of a reaction between the target substance and a ligand is limited, and an agglutination reaction is dependent on the diffusion rates of the fluorescent reagent and the antibody. That is, when the concentration of the target substance or the fluorescent reagent in the reaction liquid is low, the reaction takes time, and hence it is difficult to detect the target substance with high sensitivity within a certain period of time.

## SUMMARY OF THE INVENTION

The present invention has been made in view of such related art, and an object of the present invention is to provide a reagent and an analysis method each using particles, for enabling a specimen test based on polarization anisotropy to be performed within a short period of time and with high sensitivity.

According to one embodiment of the present invention, there is provided an analysis method including measuring a value (R) for polarization anisotropy through use of a luminescent reagent that binds with a target substance, to thereby determine at least any one of the presence or absence of the target substance and a concentration of the target substance, the analysis method including: a reaction step including mixing a sample containing the target substance with the luminescent reagent and a sensitizer, and subjecting the mixture to a reaction to obtain a reaction liquid; and a measurement step of measuring the R of the reaction liquid, the luminescent reagent including a luminescent particle substrate and a hydrophilic layer arranged on an outside of the luminescent particle substrate, the sensitizer containing a hydrophilic polymer.

In addition, according to one embodiment of the present invention, there is provided a test kit to be used for analysis involving measuring a value (R) for polarization anisotropy through use of a luminescent reagent that binds with a target substance, to thereby determine at least any one of the presence or absence of the target substance and a concentration of the target substance, the test kit including: a first reagent including a luminescent reagent including: a luminescent particle substrate; and a hydrophilic layer arranged on an outside of the luminescent particle substrate; and a second reagent including a sensitizer containing a hydrophilic polymer.

In addition, according to one embodiment of the present invention, there is provided a test reagent to be used for analysis involving measuring a value (R) for polarization anisotropy through use of a luminescent reagent that binds with a target substance, to thereby determine at least any one of the presence or absence of the target substance and a concentration of the target substance, the test reagent including: a luminescent reagent including: a luminescent particle substrate; and a hydrophilic layer arranged on an outside of the luminescent particle substrate; and a sensitizer containing a hydrophilic polymer.

Further features of the present invention will become apparent from the following description of exemplary embodiments with reference to the attached drawings.

## BRIEF DESCRIPTION OF THE DRAWINGS

FIG. 1 is a schematic view for illustrating an analysis method according to an embodiment of the present invention.

FIG. 2 is an explanatory graph of results of quantification of anti-CRP antigen concentrations through use of the analysis method according to the embodiment of the present invention.

## DESCRIPTION OF THE EMBODIMENTS

Exemplary embodiments of the present invention are described in detail below. However, the embodiments are not intended to limit the scope of the present invention.

According to one embodiment of the present invention, there is provided the following analysis method: an analysis method including measuring a value (R) for polarization anisotropy through use of a luminescent reagent that binds with a target substance, to thereby determine at least any one of the presence or absence of the target substance and a concentration of the target substance, the analysis method including: a reaction step including mixing a sample containing the target substance with the luminescent reagent and a sensitizer, and subjecting the mixture to a reaction to obtain a reaction liquid; and a measurement step of measuring the R of the reaction liquid, the luminescent reagent

including a luminescent particle substrate and a hydrophilic layer arranged on an outside of the luminescent particle substrate, the sensitizer containing a hydrophilic polymer.

(Value for Polarization Anisotropy)

In this embodiment, the value for polarization anisotropy (sometimes referred to as R) is defined as described below. That is, the R is a value showing a relationship between the luminescence intensity of a polarized light component in a parallel direction to radiated polarized light and the luminescence intensity of a polarized light component in a perpendicular direction thereto regarding luminescence generated by exciting a luminescent substance through irradiation with polarized light. More specifically, the R is a value calculated from the luminescence intensity of a luminescence component having a vibration direction parallel to that of given polarized light, the luminescence intensity being determined when the luminescent substance is excited by the polarized light. Further, the R is a value indicating the ratio of a difference between the luminescence intensity of a luminescence component having a vibration direction parallel to that of a first polarized light beam at the time of excitation by the first polarized light beam and the luminescence intensity of a luminescence component having a vibration direction orthogonal to that of the first polarized light beam at the time of excitation by the first polarized light beam to the sum of the luminescence intensities. The R may be corrected with: a ratio between the luminescence intensity of a luminescence component having a vibration direction orthogonal to that of a second polarized light beam having a vibration direction orthogonal to that of the first polarized light beam at the time of excitation by the second polarized light beam and the luminescence intensity of a luminescence component having a vibration direction parallel to that of the second polarized light beam having a vibration direction orthogonal to that of the first polarized light beam at the time of excitation by the second polarized light beam; and other constants. The value for polarization anisotropy encompasses values referred to as "polarization anisotropic property", "degree of polarization", and the like.

More specifically, for example, the R may be "r" in the following equation (1):

$$r = \frac{I_{VV} - G * I_{VH}}{I_{VV} + 2 * G * I_{VH}} \qquad G = \frac{I_{HV}}{I_{HH}} \qquad (1)$$

in the equation (1), $I_{VV}$ represents the luminescence intensity of a luminescence component having a vibration direction parallel to that of a first polarized light beam at the time of excitation by the first polarized light beam, $I_{VH}$ represents the luminescence intensity of a luminescence component having a vibration direction orthogonal to that of the first polarized light beam at the time of excitation by the first polarized light beam, $I_{HV}$ represents the luminescence intensity of a luminescence component having a vibration direction orthogonal to that of a second polarized light beam having a vibration direction orthogonal to that of the first polarized light beam at the time of excitation by the second polarized light beam, $I_{HH}$ represents the luminescence intensity of a luminescence component having a vibration direction parallel to that of the second polarized light beam having a vibration direction orthogonal to that of the first polarized light beam at the time of excitation by the second polarized light beam, and G represents a correction value.

In addition, the R may be r' in the following equation (2).

$$r' = \frac{I_{VV} - G * I_{VH}}{I_{VV} + G * I_{VH}} \qquad G = \frac{I_{HV}}{I_{HH}} \qquad (2)$$

Each symbol is the same as that in the equation (1).

With regard to conditions for the measurement of the R, for example, it is preferred that the measurement be performed in a liquid having a temperature of 0°C or more and 50°C or less, and the viscosity of the liquid be 0.5 mPa·s or more and 50 mPa·s or less. When the luminescent reagent is particles each containing a europium complex, the measurement is preferably performed with the concentration of the luminescent reagent being set to 0.001 mg/ml or more and 0.1 mg/ml or less, and an excitation wavelength is preferably 500 nm or more and 700 nm or less.

(Target Substance)

Examples of the target substance may include an antigen, an antibody, a low-molecular-weight compound, various receptors, an enzyme, a substrate, a nucleic acid, a cytokine, a hormone, a neurotransmitter, a transmitter, and a membrane protein. Examples of the antigen include an allergen, a bacterium, a virus, a cell, a cell membrane constituent, a cancer marker, various disease markers, an antibody, a blood-derived substance, a food-derived substance, a natural product-derived substance, and a low-molecular-weight compound. Examples of the nucleic acid include DNA, RNA, or cDNA derived from a bacterium, a virus, or a cell, a part or fragment thereof, a synthetic nucleic acid, a primer, and a probe. Examples of the low-molecular-weight compound include a cytokine, a hormone, a neurotransmitter, a transmitter, and a membrane protein, and receptors therefor. Examples of the antigen include a CRP antigen and a HBs antigen, and an example of the hormone is a TSH antigen.

At least any one of the presence or absence of any such target substance and the concentration of the target substance may be determined by the analysis method according to this embodiment. The presence or absence of the target substance may be determined by comparing the concentration of the target substance to a predetermined threshold. For example, the target substance may be determined to be present when the concentration of the target substance is equal to or higher than the predetermined threshold, and to be absent when the concentration is lower than the predetermined threshold.

(With regard to Reaction Step)

In the reaction step, a sample containing the target substance, the luminescent reagent, and a sensitizer are mixed, and the mixture is subjected to a reaction. In the reaction step, typically, binding between the target substance and a ligand occurs. The reaction liquid is a liquid containing the luminescent reagent, the target substance, and the sensitizer, and may further contain any other additive or the like. The reaction is performed at a pH in the range of from 3.0 or more to 11.0 or less and a temperature in the range of from 20°C or more to 50°C or less, and a reaction time may be freely decided in accordance with the detection concentration of the target substance. The target substance and the luminescent reagent are described later.

(With regard to Measurement Step)

In the measurement step, the R of the reaction liquid is measured. With regard to conditions for the measurement, for example, it is preferred that the measurement be performed in a liquid having a temperature of 0°C or more and 50°C or less, and the viscosity of the liquid be 0.5 mPa·s or more and 50 mPa·s or less. The measurement is preferably performed with the concentration of the luminescent reagent being 0.001 mg/ml or more and 0.1 mg/ml or less, and an excitation wavelength is preferably 500 nm or more and 700 nm or less.

(Sensitizer)

The sensitizer according to this embodiment contains a hydrophilic polymer. The hydrophilic polymer is preferably at least one kind selected from the group consisting of: polyvinylpyrrolidone; sodium alginate; potassium alginate; ammonium alginate; lithium alginate; alginic acid; and polyoxazoline.

The hydrophilic polymer has an effect of promoting aggregation of particles through a physical phenomenon called depletion aggregation, to thereby increase the reaction rate of the reaction between the target substance and the ligand. Description is made with reference to FIG. 1. When a distance 5 between particles of a luminescent reagent 6 is larger than the coil diameter of a hydrophilic polymer 4, the hydrophilic polymer 4 can be present between the particles. Meanwhile, when the reaction between the target substance and the ligand causes the particles of the luminescent reagent 6 to approach each other to make the distance 5 between the particles of the luminescent reagent smaller than the coil diameter of the hydrophilic polymer 4, it becomes difficult for the hydrophilic polymer 4 to be present between the particles of the luminescent reagent 6. As a result, a concentration gradient occurs in the space between the particles of the luminescent reagent and a solvent in the surroundings to generate a difference in osmotic pressure. The difference in osmotic pressure causes a force to act in the direction of aggregating the particles of the luminescent reagent 6 together. The aggregation through this force is called depletion aggregation.

With regard to conditions for causing the depletion aggregation and promoting the aggregation reaction, it is preferred that there be no interaction between the luminescent reagent 6 and the hydrophilic polymer 4. For example, in the case of an antigen-antibody reaction, the sensitizer is preferably hydrophilic as with the surface of the luminescent reagent 6. In addition, a larger molecular weight of the hydrophilic polymer 4 is advantageous because the depletion aggregation can be caused more easily even when the distance 5 between the particles of the luminescent reagent is large.

The phenomenon of depletion aggregation is dependent on the molecular weight and concentration of the

hydrophilic polymer 4, and the size of the luminescent reagent. In the case of related-art measurement based on polarization anisotropy in which the luminescent reagent is not particles and has a molecular-level size, the depletion aggregation phenomenon cannot be caused, and the reaction between the target substance and the ligand cannot be promoted. In addition, in the measurement based on polarization anisotropy, Brownian rotational motion is proportional to the viscosity of the liquid, and hence, when the liquid viscosity of the hydrophilic polymer 4 is excessively high, its molecular weight is excessively large, or its addition amount is excessively large, the R of the luminescent reagent that has not reacted (bound) with the target substance is increased.

The sensitizer is preferably hydrophilic and free from interacting with the luminescent reagent, and is preferably a hydrophilic polymer. Polyvinylpyrrolidone, an alginic acid salt, or polyoxazoline may be suitably used as the hydrophilic polymer. In addition, for the above-mentioned reason, a sensitizer having a weight-average molecular weight of about 10,000 or more and about 100,000,000 or less may be suitably used. The molecular weight is particularly preferably 100,000 or more and 5,000,000 or less, more suitably 200,000 or more and 2,000,000 or less.

An excessively small molecular weight reduces a sensitizing action. An excessively large molecular weight is not preferred because the viscosity of the solution is increased to increase the R even without the occurrence of the reaction between the target substance and the ligand. The weight-average molecular weight of the hydrophilic polymer is determined based on gel permeation chromatography (GPC) or viscosity measurement. The interaction between the hydrophilic polymer and the luminescent reagent is adjusted by selecting the hydrophilic polymer in accordance with a component of the hydrophilic layer of the luminescent reagent. In order to recognize the interaction between the sensitizer and the luminescent reagent, the R may be measured for a mixture of only the sensitizer and the luminescent reagent. The R is predicted to be increased by the effect of the increase in viscosity due to the addition of the sensitizer, but when the R is increased beyond the effect of the viscosity increase, or R is not stable, there is a risk in that the sensitizer and the luminescent reagent themselves may interact with each other. In such case, it is preferred to change the kind of the sensitizer.

The polyvinylpyrrolidone (PVP) to be used in this embodiment may be a homopolymer or a copolymer as long as the polyvinylpyrrolidone has a repeating unit represented by the chemical formula (I). Specific examples of the copolymer include copolymers of polyvinylpyrrolidone and polyethylene glycol, polyvinylpyrrolidone and polylactic acid, and polyvinylpyrrolidone and polyacrylic acid.

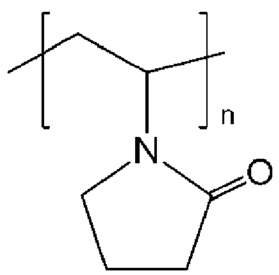

(I)

In the formula, "n" represents an integer of 100 or more.

The molecular weight of the PVP to be used as the sensitizer is preferably 100,000 or more and 5,000,000 or less, more suitably 200,000 or more and 2,000,000 or less. Preferred examples thereof include, but not limited to, PVP-K90 and PVP-130K.

The polyoxazoline to be used in this embodiment may be a homopolymer or a copolymer as long as the polyoxazoline has a repeating unit represented by the chemical formula (II). Specific examples of the copolymer include copolymers of polyoxazoline and polyethylene glycol, polyoxazoline and polylactic acid, and polyoxazoline and polyacrylic acid.

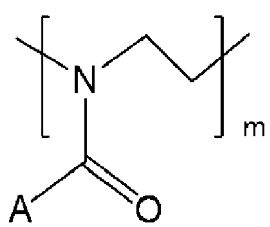

(II)

In the chemical formula (II), A's each independently represent a hydrogen atom or a methyl group, an ethyl group, or a

propyl group. "m" represents an integer of 100 or more.

The alginic acid or the alginic acid salt to be used in this embodiment is more preferably an alginic acid salt, and, for example, sodium alginate, potassium alginate, ammonium alginate, or lithium alginate may be suitably used.

In addition, the concentration of the sensitizer at the time of the measurement of the R after the reaction between the test reagent and the target substance is preferably 0.01 w/v% or more and 2.0 w/v% or less. When the concentration falls within this range, the viscosity of the solution is also low, and hence the solution is easy to handle. When the concentration of the sensitizer is excessively low, a sufficient sensitizing effect is hardly obtained. When the concentration of the sensitizer is excessively high, the reaction liquid is thickened to increase the R. Specifically, the viscosity of the solution is preferably 0.5 mPa·s or more and 15.0 mPa·s or less. When the viscosity falls within this range, the R of the luminescent reagent can be measured without being saturated even when increased by the effect of the thickening. In addition, when the viscosity of the liquid is more than 15.0 mPa·s, a risk in that air bubbles may be mixed into the sample at the time of the mixing of the reagents in the measurement process is increased. Accordingly, also from the viewpoint of handling of the reagents, the viscosity of the liquid is preferably 15.0 mPa·s or less.

In addition, the setting of the concentration may be changed depending on the kinds of the target substance to be measured and an insoluble carrier.

(Luminescent Reagent)

In this embodiment, the luminescent reagent binds with the target substance, and includes a luminescent particle substrate and a hydrophilic layer arranged on the outside of the luminescent particle substrate (i.e., the surface of the luminescent reagent).

The luminescent particle substrate contains a luminescent molecule, and the luminescent molecule is particularly preferably a molecule that is excited to emit light when irradiated with light. A molecule that emits light through a chemical reaction like luminol is not preferred. The luminescence encompasses phosphorescence and fluorescence, and is preferably phosphorescence.

In this embodiment, the luminescent particle substrate more preferably contains a europium complex.

The hydrophilic layer at the surface of the luminescent reagent preferably contains a hydrophilic polymer.

The luminescent reagent preferably includes a ligand that binds with the target substance. The "ligand" refers to a compound that specifically binds to a particular target substance. Any compound that shows affinity for a particular substance may be used as the ligand. Examples of the ligand and the target substance, or a combination of the target substance and the ligand may include the following. That is, the examples may include: an antigen and an antibody; a low-molecular-weight compound and a receptor therefor; an enzyme and a substrate; and nucleic acids complementary to each other. Further, the examples may include an antibody and any of the following substances specific thereto: an allergen, a bacterium, a virus, a cell, a cell membrane constituent, a cancer marker, various disease markers, an antibody, a blood-derived substance, a food-derived substance, a natural product-derived substance, and any low-molecular-weight compound. Further, the examples may include a receptor and any of the following substances specific thereto: a low-molecular-weight compound, a cytokine, a hormone, a neurotransmitter, a transmitter, and a membrane protein. Further, the examples may include DNA, RNA, or cDNA derived from a bacterium, a virus, or a cell, a part or fragment thereof, a synthetic nucleic acid, a primer, or a probe, and a nucleic acid having complementarity thereto. Other than the foregoing, any combination known to have affinity may be used as the combination of the target substance and the ligand. A typical example of the ligand in this embodiment is any one of an antibody, an antigen, and a nucleic acid.

The luminescent reagent is illustrated as the luminescent reagent 6 in FIG. 1, and includes a particle substrate 1 containing europium complexes 3, a hydrophilic layer 2 arranged on the outside of the particle substrate, and a ligand (not shown) that binds with the target substance. The luminescent reagent in FIG. 1 has a particulate shape, and the diameter of each of the particles is 25 nm or more and 500 nm or less.

It is preferred that the luminescent reagent to be used in this embodiment have a small particle size distribution, and the surfaces of its particles be hydrophilically coated. The europium complexes 3 are present inside the particles.

The diameter of each of the particles may be determined by a dynamic light scattering method. When particles dispersed in a solution are irradiated with laser light and the resultant scattered light is observed with a photon detector, an intensity distribution due to interference of the scattered light is constantly fluctuating because the particles are constantly shifting their positions by Brownian motion.

The dynamic light scattering method is a measurement method for observing the state of the Brownian motion as a fluctuation in scattered light intensity. The fluctuation of scattered light with respect to time is expressed as an autocorrelation function, and a translational diffusion coefficient is determined. A Stokes diameter is determined from the determined diffusion coefficient, and the size of each of the particles dispersed in the solution can be derived.

The luminescent reagent desirably has nothing provided on the surfaces of its particles from the viewpoint of keeping the uniformity and monodispersity of the particles. However, for the purpose of use in the analysis method according to this embodiment, nonspecific adsorption of substances other than the target onto the particles needs to be

prevented, and hence the luminescent reagent includes the hydrophilic layer at its surface in order to keep the surface hydrophilic.

For the hydrophilic layer at the surface, a method involving supporting BSA on the surface of each of the particles is widely used as a technique for keeping hydrophilicity, but this method may cause a lot variation. In view of this, the luminescent reagent preferably includes a hydrophilic layer having a hydrophilic polymer. The concentration of the luminescent reagent in the reaction liquid is preferably 0.000001 mass% or more and 1 mass% or less, more preferably 0.00001 mass% or more and 0.001 mass% or less.

The luminescent reagent to be used in this embodiment can emit phosphorescence having a long lifetime by virtue of containing the europium complex. When the luminescent reagent to be used in this embodiment has a particulate shape, an average particle diameter that is the average of the diameters of the particles is preferably 25 nm or more and 500 nm or less, and the average particle diameter is more preferably 50 nm or more and 300 nm or less. When the average particle diameter is more than 500 nm, the polarization anisotropic property before aggregation becomes high, resulting in a small difference from the polarization anisotropic property after the aggregation reaction. In addition, when the average particle diameter is less than 25 nm, a change between sizes before and after the aggregation becomes small to make it difficult to grasp the change of the R through phosphorescent luminescence depolarization.

By reducing the particle size distribution of the luminescent reagent and introducing the europium complex as the luminescent molecule, a change in polarized luminescence characteristic can be grasped even when the dispersion state of the particles in the liquid undergoes a slight change. Specifically, even if the concentration of the target substance in the solution is from about a nanogram to about a picogram per mL, when the luminescent reagent aggregates via the target substance, a change in rotational Brownian motion of the luminescent reagent can be grasped as a change in polarization anisotropy.

The "polarized luminescence" refers to the following phenomenon: when a luminescent material having an anisotropic property in transition moment (transition dipole moment) uses polarized light along its transition moment as excitation light, its luminescence is also polarized light along the transition moment. The europium complex shows fluorescent luminescence based on energy transfer from the ligand to the central metal ion, and hence the transition moment is complicated, but red luminescence around 610 nm, which is derived from electronic transition from the lowest excited state 5D0 to 7F2, is emitted as polarized light.

The principle of polarization anisotropy is the measurement of a shift in transition moment due to the rotational motion of a luminescent material during the occurrence of polarized luminescence. The rotational motion of the luminescent material may be expressed by the equation (3):

$$Q=3V\eta/kT \cdots (3)$$

where Q represents the rotational relaxation time of the material, V represents the volume of the material, $\eta$ represents the viscosity of a solvent, k represents the Boltzmann constant, and T represents an absolute temperature.

The rotational relaxation time of the material is a period of time required for a molecule to rotate by an angle $\theta$ (68.5°) at which $\cos\theta=1/e$.

It is found from the equation (3) that the rotational relaxation time of the luminescent material is proportional to the volume of the material, that is, when the luminescent material has a particulate shape, the cube of the particle diameter. Meanwhile, a relationship between the emission lifetime of the luminescent material and the degree of polarization serving as the value for polarization anisotropy may be expressed by the equation (4):

$$p0/p=1+A(\tau/Q) \cdots (4)$$

where p0 represents a degree of polarization at a time when the material is stationary ($Q=\infty$), "p" represents the degree of polarization, A is a constant, $\tau$ represents the emission lifetime of the material, and Q represents the rotational relaxation time.

It is found from the equation (3) and the equation (4) that the degree of polarization is influenced by the emission lifetime of the luminescent material and the rotational relaxation time, that is, the volume (particle diameter) of the luminescent material, i.e., influenced by the balance between the particle diameter and emission lifetime of the luminescent material.

When the degree of polarization of the luminescent material represented by the equation (4) is determined experimentally, it is appropriate that polarized light be allowed to enter the luminescent material, and luminescence be detected in a 90° direction with respect to the traveling direction and vibration direction of excitation light. In this case, it is appropriate that the detected light be detected by being divided into polarized light components in parallel and perpendicular directions with respect to the polarized light that is the incident light, and for example, a polarization

anisotropic property represented by the equation (5) be adopted as the value for polarization anisotropy:

$$R(t)=(I/\!/(t)-GI\perp(t))/(I/\!/(t)+2GI\perp(t)) \cdots (5)$$

where R(t) represents a polarization anisotropic property at a time "t", I//(t) represents the luminescence intensity of a luminescence component parallel to the excitation light at the time "t", I⊥(t) represents the luminescence intensity of a luminescence component perpendicular to the excitation light at the time "t", and G represents a correction value, the ratio of I⊥/I// measured with excitation light having a vibration direction different by 90° from that of the excitation light used for sample measurement.

That is, when the particle size and the emission lifetime fall within appropriate ranges, a change in size of the luminescent material due to, for example, a reaction with the target substance can be sensitively read as a change in polarization anisotropic property. That is, the r(t) of the unaggregated luminescent material is observed to be low, and the r(t) of the aggregated luminescent material is observed to be high. This is the principle of polarization anisotropy.

The value for polarization anisotropy may be corrected with G and 2G, or may be a value without G and 2G.

(Particle Substrate 1)

FIG. 1 is an illustration of an example of the luminescent reagent 6 having a particulate shape (spherical shape), and the luminescent reagent 6 includes the particle substrate 1. In FIG. 1, an example in which the luminescent reagent 6 and the particle substrate 1 both have spherical shapes is illustrated, but the shapes of the luminescent reagent 6 and the particle substrate 1 in this embodiment are not limited. The particle substrate 1 is not particularly specified as long as the particle substrate 1 is a material capable of stably incorporating the europium complex, but is preferably a polymer containing a styrene unit and an organic silane unit. In particular, for example, a polymer obtained by polymerizing a composition containing styrene as a main component and a radically polymerizable organic silane is suitably used. When the composition contains styrene as the main component, particles having an extremely uniform particle size distribution can be produced by an emulsion polymerization method to be described later. In addition, when a polymer containing an organic silane unit is adopted, a silanol group (Si-OH) is produced in the polymer in an aqueous solvent, and particle substrate surfaces form a siloxane bond (Si-O-Si) to each other, via which the hydrophilic layer to be described later or a ligand can be provided. The particles according to this embodiment each preferably have a ligand-bonding functional group capable of bonding a ligand to the outside of the particle substrate.

(Hydrophilic Layer 2)

The hydrophilic layer 2 may be formed by incorporating a hydrophilic polymer or a hydrophilic molecule on the outside of the particle substrate 1. The hydrophilic polymer or the hydrophilic molecule is a polymer or molecule containing a hydrophilic group, and specific examples of the hydrophilic group include molecules or polymers each having a hydroxy group, an ether, pyrrolidone, or a betaine structure. Specific examples of the hydrophilic polymer include polyethylene glycol, polyvinylpyrrolidone, a polymer of sulfobetaine, a polymer of phosphobetaine, and polyglycidyl methacrylate whose molecule has an end modified with a hydroxy group by ring-opening a glycidyl group, and those hydrophilic polymers may each be used as a main component of the hydrophilic layer 2. Alternatively, the hydrophilic layer 2 may be formed by directly providing a single molecule having a hydrophilic group on the surface of the particle substrate 1 through use of a silane coupling agent or the like. The thickness of the hydrophilic layer 2 is not limited, but does not need to be set to be large beyond a thickness with which hydrophilicity can be exhibited. When the hydrophilic layer 2 is excessively thick, there is a risk in that the hydrophilic layer may become hydrogel-like and be hydrated by the influence of ions in the solvent, to thereby make its thickness unstable. The thickness of the hydrophilic layer 2 is suitably 1 nm or more and 15 nm or less.

(Europium Complex 3)

The europium complex 3 has a feature in that the wavelength and intensity of its luminescence are hardly influenced by the surroundings, and hence the luminescence has a long lifetime. The europium complex 3 is made up of a europium element and a ligand. In consideration of the emission lifetime, a visible emission wavelength region, and the like, a luminescent dye is preferably a europium complex. Europium generally has a emission lifetime of 0.1 ms or more and 1.0 ms or less. The emission lifetime and the rotational relaxation time obtained from the equation (1) need to be appropriately adjusted. In the case of europium in a water dispersion, when the diameter of the luminescent reagent is 50 nm or more and 300 nm or less, the R significantly changes before and after aggregation.

At least one of the constituent ligands of the europium complex 3 is a ligand having a light-collecting function.

The "light-collecting function" refers to an action of being excited at a particular wavelength to excite the central metal of the complex through energy transfer. In addition, it is preferred that the constituent ligands of the europium complex 3 include a ligand such as a β-diketone to prevent coordination of a water molecule. The ligand such as the β-diketone coordinated to a europium ion suppresses a deactivation process due to the transfer of energy to a solvent molecule or the like to provide strong luminescence.

The europium complex 3 may be a polynuclear complex.

In addition, specific examples of the europium complex include [tris(2-thenoyltrifluoroacetone)(bis(triphenylphosphineoxide))europium(III)], [tris(2-thenoyltrifluoroacetone)(triphenylphosphineoxide)(dibenzylsulfoxide)europium(III)], and [tris(2-thenoyltrifluoroacetone)(phenanthroline)europium(III)].

At the time of a state in which the Brownian rotational motion of the europium complex 3 can be regarded as stationary in a medium, the polarization anisotropic property expressed by the equation (3) is desirably 0.08 or more. The state in which the Brownian rotational motion can be regarded as stationary refers to a state in which the rotational relaxation time of the particles is sufficiently longer than the emission lifetime of the europium complex 3.

The europium complex 3 is preferably incorporated in a larger amount into the particle substrate 1 because a luminescence intensity per particle becomes stronger. Meanwhile, when the europium complexes 3 aggregate in the particle substrate 1, an interaction between ligands influences the excitation efficiency of the europium complex 3 and the like to make it difficult to measure the polarization anisotropic property while keeping reproducibility. Whether the europium complex 3 shows non-aggregated luminescence behavior in the particle substrate 1 may be judged from an excitation spectrum of the sample.

Particles having strong luminescence not only enable high-sensitivity measurement, but also enable an increase in biochemical reaction rate because luminescence is kept even when their particle diameters are reduced. As the particle diameters become smaller, the diffusion coefficient of Brownian motion in the liquid becomes larger, and hence the reaction can be detected in a shorter period of time.

(Method of producing Luminescent Reagent)

Next, an example of a method of producing the luminescent reagent to be used in this embodiment is described.

The method of producing the luminescent reagent includes a step (first step) of mixing radically polymerizable monomers including at least styrene and a radically polymerizable organic silane, a radical initiator, a polarized luminescent europium complex, and a hydrophilic polymer with an aqueous medium to prepare an emulsion.

Further, the method of producing the luminescent reagent includes a step (second step) of heating the emulsion to polymerize the radically polymerizable monomers.

The method of producing the luminescent reagent may include a step (third step) of providing a ligand-bonding functional group to be described later on the surface of the luminescent reagent. Herein, the ligand-bonding functional group refers to a functional group that can bond a ligand. Specifically, any one of a carboxy group, an amino group, a thiol group, an epoxy group, a maleimide group, a succinimidyl group, or an alkoxysilyl group (silicon alkoxide structure) may be used.

(Radically Polymerizable Monomers)

The production of the luminescent reagent is performed by polymerizing radically polymerizable monomers, and the radically polymerizable monomers include at least styrene and a radically polymerizable organic silane. The radically polymerizable monomers may further include at least one kind of monomer selected from the group consisting of: an acrylate-based monomer; and a methacrylate-based monomer. Examples of the monomer may include butadiene, vinyl acetate, vinyl chloride, acrylonitrile, methyl methacrylate, methacrylonitrile, methyl acrylate, and mixtures thereof. That is, one kind or a plurality of kinds of those monomers may be used in addition to styrene and the radically polymerizable organic silane. In addition, a monomer having two or more double bonds per molecule, such as divinylbenzene, may be used as a crosslinking agent.

The inclusion of the radically polymerizable organic silane in the radically polymerizable monomers provides a siloxane bond on the particle substrate 1. Examples of the radically polymerizable organic silane may include vinyltrimethoxysilane, vinyltriethoxysilane, p-styryltrimethoxysilane, 3-methacryloxypropylmethyldimethoxysilane, 3-methacryloxypropyltrimethoxysilane, 3-methacryloxypropylmethyldiethoxysilane, 3-methacryloxypropyltriethoxysilane, 3-acryloxypropyltrimethoxysilane, and combinations thereof. The use of the radically polymerizable organic silane serves to form a backbone of an inorganic oxide in the particle substrate 1 to improve the physical and chemical stability of the luminescent reagent. Further, the use of the radically polymerizable organic silane enhances affinity between the particle substrate 1 and each of the hydrophilic layer 2 and the ligand-bonding functional group.

Further, the inclusion of the radically polymerizable organic silane in the radically polymerizable monomers provides a silanol group on the surface of the particle substrate 1. The silanol group and the hydrophilic polymer such

as PVP form a hydrogen bond. Thus, the hydrophilic polymer such as PVP is more strongly adsorbed onto the surface of the particle substrate 1.

(Radical Initiator)

A wide range of compounds selected from, for example, azo compounds and organic peroxides may each be used as the radical initiator. Specific examples thereof may include 2,2'-azobis(isobutyronitrile), 2,2'-azobis(2,4-dimethylvaleronitrile), 2,2'-azobis(2-methylbutyronitrile), 4,4'-azobis(4-cyanovaleric acid), 2,2'-azobis(2-methylpropionamidine) dihydrochloride, dimethyl 2,2'-azobis(2-methylpropionate), tert-butyl hydroperoxide, benzoyl peroxide, ammonium persulfate (APS), sodium persulfate (NPS), and potassium persulfate (KPS).

(Hydrophilic Polymer)

The luminescent reagent may include a hydrophilic polymer as the hydrophilic layer. The hydrophilic polymer preferably suppresses nonspecific adsorption. Examples of the hydrophilic polymer include hydrophilic polymers each containing a unit having an ether, a betaine, or a pyrrolidone ring. It is preferred that the hydrophilic layer be included in the synthesized luminescent reagent, and be mainly present on the particle surface on the outside of the particle substrate. Herein, a polymer having a pyrrolidone ring is sometimes abbreviated as "PVP". When the PVP is fed at the time of the synthesis of the luminescent reagent, a nonspecific adsorption-suppressing ability and a ligand-bonding ability can be simultaneously provided for the luminescent reagent. The PVP to be fed at the time of the synthesis has higher hydrophilicity than the radically polymerizable monomers, and hence is present at an interface between the solvent and the particle substrate that is being polymerized at the time of the synthesis. The particle substrate 1 adsorbs the PVP onto the outside thereof by involving part of the PVP at the time of the polymerization, or by physical/chemical adsorption such as an interaction between a pyrrolidone ring and styrene (radically polymerizable monomer).

The molecular weight of the PVP is preferably 10,000 or more and 100,000 or less, more suitably 40,000 or more and 70,000 or less. When the molecular weight is less than 10,000, the hydrophilicity of the surface of the luminescent reagent is weak, and hence nonspecific adsorption is liable to occur. When the molecular weight is more than 100,000, the hydrophilic layer becomes so thick as to gel, thereby becoming difficult to handle.

In addition to the PVP, another hydrophilic polymer may be added as a protective colloid at the time of the synthesis of the particle substrate.

In addition, the luminescent reagent preferably satisfies $A2-A1 \leq 0.1$.

A1 and A2 are defined as described below. That is, with regard to a mixture obtained by adding 30 $\mu$L of a 0.1 wt% dispersion of the luminescent reagent to 60 $\mu$L of a buffer solution mixed with 16 $\mu$L of human serum diluted 15-fold, the absorbance of the mixture immediately after the addition is represented by A1, and the absorbance of the mixture after being left to stand at 37°C for 5 minutes after the addition is represented by A2. The absorbances are measured at an optical path of 10 mm and a wavelength of 572 nm.

Particles showing an A2-A1 of 0.1 or less have little nonspecific adsorption of impurities in serum, and hence are preferred.

(Aqueous Medium)

The aqueous medium (aqueous solution) to be used for the above-mentioned method of producing the luminescent reagent preferably contains water at 80 wt% or more and 100 wt% or less in the medium. The aqueous solvent is preferably water or a watersoluble organic solvent, and examples thereof include solutions each obtained by mixing water with methanol, ethanol, isopropyl alcohol, or acetone. When an organic solvent other than water is incorporated at more than 20 wt%, there is a risk in that dissolution of the polymerizable monomers may occur at the time of the production of the particles.

In addition, the aqueous medium preferably has its pH adjusted to 6 or more and 9 or less in advance. When the pH has a value of less than 6 or more than 9, there is a risk in that an alkoxide group or silanol group of the radically polymerizable organic silane may undergo condensation polymerization or a reaction with another functional group before the formation of the polymer, leading to aggregation of the particles to be obtained. In this embodiment, the alkoxide is not intentionally subjected, before the polymerization, to condensation polymerization.

The above-mentioned pH is preferably adjusted using a pH buffer, but may be adjusted with an acid or a base.

Other than the foregoing, a surfactant, an antifoaming agent, a salt, a thickener, and the like may be used by being added at a ratio of 10% or less with respect to the aqueous medium.

In the production of the luminescent reagent, it is preferred that, first, the PVP be dissolved in the aqueous medium whose pH has been adjusted to 6 or more and 9 or less. The content of the PVP is preferably 0.01 wt% or more and 10 wt% or less, more preferably 0.03 wt% or more and 5 wt% or less with respect to the aqueous medium. When

the content is less than 0.01 wt%, the amount of adsorption onto the particle substrate is small, and the effect thereof is hardly expressed. In addition, when the content is more than 10 wt%, there is a risk in that the viscosity of the aqueous medium may be increased to preclude sufficient stirring.

Subsequently, the radically polymerizable monomers including the styrene (A) and the radically polymerizable organic silane (B) are added into the above-mentioned aqueous medium to prepare an emulsion. A weight ratio between the styrene (A) and the radically polymerizable organic silane (B) is from 6:4 to 100:1. Further, the prepared emulsion is mixed with the europium complex. At this time, when the solubility of the europium complex is low, a water-insoluble organic solvent may be added. A weight ratio between the europium complex and the radically polymerizable monomers is from 1:1,000 to 1:10.

When the weight ratio between the styrene (A) and the radically polymerizable organic silane (B) is less than 6:4, there is a risk in that the specific gravity of the particles as a whole may be increased, resulting in remarkable sedimentation of the particles. In addition, in order to increase adhesiveness between the PVP and luminescent particles, it is desired that the weight ratio between the styrene (A) and the radically polymerizable organic silane (B) be set to 100: 1 or more.

A weight ratio between the weight of the aqueous medium and the total amount of the radically polymerizable monomers is preferably from 5:5 to 9.5:0.5. When the weight ratio between the weight of the aqueous medium and the total amount of the radically polymerizable monomers is less than 5:5, there is a risk in that remarkable aggregation of the particles to be produced may occur. In addition, when the weight ratio between the weight of the aqueous medium and the total amount of the radically polymerizable monomers is more than 9.5:0.5, although there is no problem with the production of the particles, there is a risk in that the production amount thereof may be reduced.

The radical initiator is used by being dissolved in water, a buffer, or the like. The radical initiator may be used between 0.5 mass% or more and 10 mass% or less in the emulsion with respect to the total weight of the styrene (A) and the radically polymerizable organic silane (B).

In the above-mentioned step of heating the emulsion, it is only required that the entire emulsion be uniformly heated. A heating temperature may be arbitrarily set between 50°C or more and 80°C or less, and a heating time may be arbitrarily set between 2 hours or more and 24 hours or less. Through the heating of the emulsion, the radically polymerizable monomers are polymerized.

The luminescent reagent may have a ligand-bonding functional group on its surface. The ligand-bonding functional group is not particularly limited as long as the functional group can bond an antibody, an antigen, an enzyme, or the like, but for example, may be a carboxy group, an amino group, a thiol group, an epoxy group, a maleimide group, a succinimidyl group, a silicon alkoxide group, or the like, or contain any of those functional groups. For example, a silane coupling agent having the ligand-bonding functional group and the synthesized particles may be mixed to provide the functional group on the particle surface. Specifically, an aqueous solution of a silane coupling agent having a carboxy group may be prepared and mixed with a dispersion of the synthesized particles to provide the carboxy group on the particle surface. At this time, a dispersant such as Tween 20 may be added to the reaction solution. A reaction temperature may be arbitrarily set between 0°C or more and 80°C or less, and a reaction time may be arbitrarily set between 1 hour or more and 24 hours or less. In order to suppress an abrupt condensation reaction of the silane coupling agent, it is suitable that the temperature be set to be equal to or lower than a room temperature of about 25°C, and the reaction time be set to 3 hours or more and 14 hours or less. Depending on the ligand-bonding functional group, the reaction with the particle surface may be promoted by adding an acid or alkali catalyst.

The luminescent reagent can be utilized as particles for a specimen test by bonding a ligand such as any of various antibodies thereto. An optimal technique for bonding an antibody of interest or the like through utilization of a functional group present on the hydrophilic layer 2 only needs to be selected.

(Introduction of Ligand)

A hitherto known method may be applied to a chemical reaction for chemically bonding the ligand-bonding functional group and the ligand to the extent that the object of the present invention can be achieved. In addition, when the ligand is amide-bonded, a catalyst such as 1-[3-(dimethylaminopropyl)-3-ethylcarbodiimide] may be appropriately used.

The luminescent reagent to be used in this embodiment may be preferably applied to a latex immunoagglutination measurement method utilized widely in the fields of clinical tests, biochemical research, and the like.

(Test Reagent)

According to a further embodiment of the present invention, there is provided a test reagent to be used for analysis involving measuring a value (R) for polarization anisotropy through use of a luminescent reagent that binds with a target substance, to thereby determine at least any one of the presence or absence of the target substance and

a concentration of the target substance, the test reagent including: a luminescent reagent including: a luminescent particle substrate; and a hydrophilic layer arranged on an outside of the luminescent particle substrate; and a sensitizer containing a hydrophilic polymer.

In the test reagent of this embodiment, the hydrophilic polymer is preferably at least one selected from the group consisting of: polyvinylpyrrolidone; sodium alginate; potassium alginate; ammonium alginate; lithium alginate; alginic acid, and polyoxazoline. In addition, the hydrophilic polymer has a molecular weight of 200,000 or more and 2,000,000 or less. It is preferred that a luminescent molecule be a europium complex. The luminescent reagent preferably includes a ligand that binds with the target substance. In addition, it is preferred that $R0 \geq 0.001$, where R0 represents the R measured for the luminescent reagent unreacted with the target substance.

The test reagent in this embodiment is used for the analysis of a target substance in a sample in *in vitro* diagnosis.

The test reagent may include the luminescent reagent, the sensitizer, and further, a dispersion medium. The amount of the luminescent reagent according to this embodiment in the test reagent is preferably 0.000001 mass% or more and 20 mass% or less, more preferably 0.0001 mass% or more and 1 mass% or less. The amount of the hydrophilic polymer sensitizer according to this embodiment in the test reagent in this embodiment is preferably 0.01 w/v% or more and 2.0 w/v% or less. The test reagent according to this embodiment may include, in addition to the luminescent reagent according to this embodiment, a third substance, such as an additive or a blocking agent, to the extent that the object of the present invention can be achieved. The test reagent may include a combination of two or more kinds of third substances, such as an additive and a blocking agent. Examples of the dispersion medium to be used in this embodiment include various buffer solutions, such as a phosphate buffer solution, a glycine buffer solution, a Good's buffer solution, a Tris buffer solution, and an ammonia buffer solution, but the dispersion medium included the test reagent in this embodiment is not limited thereto. The test reagent in this embodiment may be stored as test reagents in which the components are each independently present. The test reagents in which the components are each independently present may be mixed at the time of measurement to prepare a test reagent containing components needed for a specimen test.

When the test reagent in this embodiment is used for the detection of an antigen or an antibody in a specimen, an antibody or an antigen may be used as the ligand.

(Test Kit)

According to a further embodiment of the present invention, there is provided a test kit to be used for analysis involving measuring a value (R) for polarization anisotropy through use of a luminescent reagent that binds with a target substance, to thereby determine at least any one of the presence or absence of the target substance and a concentration of the target substance, the test kit including: a first reagent including a luminescent reagent including: a luminescent particle substrate; and a hydrophilic layer arranged on an outside of the luminescent particle substrate; and a second reagent including a sensitizer containing a hydrophilic polymer.

In the test kit of this embodiment, the hydrophilic polymer is preferably at least one selected from the group consisting of: polyvinylpyrrolidone; sodium alginate; potassium alginate; ammonium alginate; lithium alginate; alginic acid; and polyoxazoline. In addition, the hydrophilic polymer has a molecular weight of 200,000 or more and 2,000,000 or less. It is preferred that a luminescent molecule be a europium complex. The luminescent reagent preferably includes a ligand that binds with the target substance. In addition, it is preferred that $R0 \geq 0.001$, where R0 represents the R measured for the luminescent reagent unreacted with the target substance.

The test kit in this embodiment is used for the analysis of a target substance in a sample in *in vitro* diagnosis.

The first reagent and the second reagent may each include a dispersion medium. The test reagent according to this embodiment may include, in addition to the luminescent reagent according to this embodiment, a third substance, such as an additive or a blocking agent, to the extent that the object of the present invention can be achieved. The test reagent may include a combination of two or more kinds of third substances, such as an additive and a blocking agent. Examples of the dispersion medium to be used in this embodiment include various buffer solutions, such as a phosphate buffer solution, a glycine buffer solution, a Good's buffer solution, a Tris buffer solution, and an ammonia buffer solution, but the dispersion medium to be incorporated into the test reagent in this embodiment is not limited thereto.

The first reagent and the second reagent are mixed with a sample containing the target substance to be used for analysis involving measuring the value (R) for polarization anisotropy, to thereby determine at least any one of the presence or absence of the target substance and the concentration of the target substance. The order of mixing is not limited, and the second reagent of this kit may be used by being mixed with the first reagent, or may be used by being mixed with the sample containing the target substance, or the second reagent may be used by being mixed after the sample containing the target substance and the first reagent have been mixed.

The concentration of the first reagent is preferably adjusted so that the amount of the luminescent reagent in the mixed liquid is 0.000001 mass% or more and 20 mass% or less, more preferably 0.0001 mass% or more and 1 mass% or less. In addition, the concentration of the second reagent is preferably adjusted so that the amount of the sensitizer in the mixed liquid is 0.01 w/v% or more and 2.0 w/v% or less.

The test kit may further include a container containing the first reagent or the second reagent, and a case enclosing the container. The first reagent and the second reagent may each be diluted as appropriate. In addition, the test kit may further include a positive control, a negative control, a diluent, or the like. As a medium for the positive control or the negative control, there may be used serum free of a measurable target substance, physiological saline, or a solvent.

[Examples]

The present invention is specifically described below by way of Examples. However, the present invention is not limited to these Examples.

(1) Production of Luminescent Particles

Polyvinylpyrrolidone (PVP-K30: manufactured by Tokyo Chemical Industry Co., Ltd.) was dissolved in a 2-morpholinoethanesulfonic acid (MES) buffer solution (manufactured by Kishida Chemical Co., Ltd.) having a pH of 7 to prepare a solvent A. [Tris(2-thenoyltrifluoroacetone)(bis(triphenylphosphineoxide))europium(III)] (manufactured by Central Techno Corporation, hereinafter abbreviated as "$Eu(TTA)_3(TPPO)_2$") serving as a europium complex, a styrene monomer (manufactured by Kishida Chemical Co., Ltd.), 3-methacryloxypropyltrimethoxysilane (manufactured by Tokyo Chemical Industry Co., Ltd., hereinafter abbreviated as "MPS") were mixed to prepare a reaction liquid B. The reaction liquid B was added into a four-necked flask containing the solvent A, and the mixture was stirred with a mechanical stirrer set to 300 rpm. After 15 minutes of stirring under a nitrogen flow condition, the temperature of an oil bath that had been prepared was set to 70°C, and the nitrogen flow was performed for an additional 15 minutes. After the mixed liquid had been heated and stirred, an aqueous solution having dissolved therein potassium persulfate (hereinafter abbreviated as "KPS") (manufactured by Sigma-Aldrich) was added into the reaction solution, and emulsion polymerization was performed for 20 hours. After the polymerization reaction, the resultant suspension was subjected to ultrafiltration with about 4 L of ion-exchanged water through use of an ultrafiltration membrane having a molecular weight cutoff of 100K to wash the product, to thereby provide a dispersion of luminescent particles.

An aliquot of the dispersion of the luminescent particles obtained by the emulsion polymerization was taken and added to an aqueous solution having dissolved therein 1 mass% of Tween 20 (manufactured by Kishida Chemical Co., Ltd.). After 10 minutes of stirring, a silane coupling agent, X12-1135 (manufactured by Shin-Etsu Chemical Co., Ltd.), was added, and the mixture was stirred overnight. After the stirring, the dispersion was centrifuged, the supernatant was removed, and the precipitate was redispersed with pure water. The operations of centrifugation and redispersion were performed 3 or more times to wash the product. The precipitate after the washing was redispersed in pure water. Thus, ligand-bonding functional groups were introduced into particles 1 to 8. A mass ratio among the particles, pure water, and X12-1135 loaded was set to 1:300:2.

(Production of Luminescent Reagent having anti-CRP Antibody)

An aliquot of 0.25 mL of the particle dispersion at 1.2 wt% corresponding to synthesized luminescent particles was taken, and the solvent was replaced by 1.6 mL of a MES buffer solution having a pH of 6.0. To the particle MES buffer solution, 1-[3-(dimethylamino)propyl]-3-ethylcarbodiimide and N-hydroxysulfosuccinimide sodium were added at 0.5 wt%, and the mixture was subjected to a reaction at 25°C for 1 hour. After the reaction, the dispersion was washed with a MES buffer solution having a pH of 5.0, an anti-CRP antibody was added at 100 μg/mL, and the anti-CRP antibody was bonded to the particles at 25°C for 2 hours. After the bonding, the particles were washed with a Tris buffer solution having a pH of 8. After the reaction, the particles were washed with a phosphate buffer solution to provide an anti-CRP antibody-modified luminescent reagent having a concentration of 0.3 wt% (sometimes referred to as "affinity particles").

(Production of Luminescent Reagent having anti-TSH Antibody)

An aliquot of 0.25 mL of the particle dispersion at 1.2 wt% corresponding to synthesized luminescent particles was taken, and the solvent was replaced by 1.6 mL of a MES buffer solution having a pH of 6.0. To the particle MES buffer solution, 1-[3-(dimethylamino)propyl]-3-ethylcarbodiimide and N-hydroxysulfosuccinimide sodium were added at 0.5 wt%, and the mixture was subjected to a reaction at 25°C for 1 hour. After the reaction, the dispersion was washed with a MES buffer solution having a pH of 5.0, an anti-TSH antibody was added at 100 μg/mL, and the anti-TSH antibody was bonded to the particles at 25°C for 2 hours. After the bonding, the particles were washed with a Tris buffer solution having a pH of 8. After the reaction, the particles were washed with a phosphate buffer solution to provide an anti-TSH antibody-modified luminescent reagent having a concentration of 1.0 wt% (sometimes referred to as "affinity particles"). The anti-TSH antibodies used were monoclonal antibodies, and the luminescent particles were modified with two kinds of anti-TSH antibodies in order to cause at least two or more particles to react with a TSH antigen serving as a measurement

object.

The bonding of the antibodies to the particles was recognized by measuring the amount of a reduction in antibody concentration in the buffer solution having added thereto the antibodies by BCA assay.

(Preparation of Luminescent Reagent Liquid)

The resultant luminescent reagent was diluted with a phosphate (PBS) buffer solution having a pH of 7.4 so as to have a concentration of 0.1 mg/mL to prepare a luminescent reagent liquid.

(Preparation of Diluted Liquid)

A 4-(2-hydroxyethyl)-1-piperazineethanesulfonic acid (HEPES) buffer solution and a PBS buffer solution were mixed at a ratio of 1:1 in terms of volume ratio, and a sensitizer was appropriately added to give a diluted liquid. The kind and amount of the sensitizer added are shown in Examples to be described later.

Reaction between Target Substance and Ligand (Antigen-antibody Reaction)

A liquid obtained by mixing a CRP antigen into a HEPES buffer solution was prepared, and was warmed to a temperature of 37°C. The luminescent reagent liquid was added to the prepared liquid, and the whole was quickly stirred, followed by observation of the polarization anisotropy of the mixed liquid. In each of Examples 1, 2, 3, and 4, and Comparative Example, an investigation was performed using the luminescent reagent at 0.01 mg/mL and CRP at an antigen concentration of 200 pM. In Example 5, an investigation was performed using the luminescent reagent at 0.0025 mg/mL and CRP at an antigen concentration of 1 pM. In Example 6, an investigation was performed using the luminescent reagent at 0.04 mg/mL and TSH at an antigen concentration of 100 pM. The observation was performed at a temperature of 37°C. The polarization anisotropy is described later.

(Example 1)

Sodium Alginate 80-120 (manufactured by FUJIFILM Wako Pure Chemical Corporation) was added as the sensitizer at a final concentration of 0.1 w/v%, and the mixture was subjected to the antigen-antibody reaction and measurement of fluorescence polarization. Based on the results of the measurement, evaluation of the CRP antigen concentration was performed.

(Example 2)

Evaluation of the CRP antigen concentration was performed by the same method as in Example 1 except that 0.2 w/v% of PVP-K90 (manufactured by Tokyo Chemical Industry Co., Ltd.: molecular weight: 360,000) was used as the sensitizer.

(Example 3)

Evaluation of the CRP antigen concentration was performed by the same method as in Example 1 except that 0.4 w/v% of PVP-K90 (manufactured by Tokyo Chemical Industry Co., Ltd.: molecular weight: 360,000) was used as the sensitizer.

(Example 4)

Evaluation of the CRP antigen concentration was performed by the same method as in Example 1 except that 0.1 w/v% of PVP 1300K (manufactured by Merck: molecular weight: 1,300,000) was used as the sensitizer.

(Example 5)

Sodium Alginate 80-120 (manufactured by FUJIFILM Wako Pure Chemical Corporation) and polyethylene glycol (hereinafter abbreviated as PEG) (manufactured by FUJIFILM Wako Pure Chemical Corporation: molecular weight: 500,000) were added as the sensitizer at final concentrations of 0.2 w/v% and 0.2 w/v%, respectively, and the mixture was subjected to the antigen-antibody reaction and measurement of fluorescence polarization. Based on the results of the measurement, evaluation of the CRP antigen concentration was performed.

(Example 6)

Poly(2-ethyl-2-oxazoline) (manufactured by Sigma-Aldrich: molecular weight: 500,000) was added as the sensitizer at a final concentration of 2.0 w/v%, and the mixture was subjected to the antigen-antibody reaction and measurement of fluorescence polarization. Based on the results of the measurement, evaluation of the TSH antigen concentration was performed.

(Comparative Example 1)

Evaluation of the CRP antigen concentration was performed by the same method as in Example 1 except that no sensitizer was used.

(Evaluations of Products)

The products in Examples and Comparative Example were each subjected to such evaluations as described below.

The shape of the product was evaluated using an electron microscope (S5500 manufactured by Hitachi High-Technologies Corporation).

The average particle diameter of the product was evaluated using dynamic light scattering (Zetasizer Nano S manufactured by Malvern).

The concentration of a suspension having the product dispersed therein was evaluated using a gravimetric analyzer (Thermo plus TG8120 manufactured by Rigaku Corporation).

Measurement of R was performed using the following devices.

An LED light source of excitation light at 340 nm was prepared, and a polarizing filter (manufactured by Sigmakoki Co., Ltd., NSPFU-30C) and a shortpass filter (manufactured by Edmund Optics, 84-706) were inserted into an optical path to set an optical system capable of irradiating a 1 cm quartz square cell. A polarizing filter (manufactured by Thorlabs, Inc., PIVISC050) and a bandpass filter (manufactured by Thorlabs, Inc., FB610-10) were set in a direction of 90° with respect to incident light. In order to measure luminescence as $I_{VV}$ and $I_{VH}$ in two directions at the same time, two sets in which the construction of a polarizer was changed by a direction of 90° with respect to incident light were prepared. For the detection of polarized light, spectrometry was performed using QEPro manufactured by Ocean Optics, Inc. Temperature control was set for a sample holder so as to enable measurement at 37°C. Measurement of the polarization anisotropic property "r" was performed with the LED light source being fixed at an output of 12 mW and a cumulative time being set to 3 seconds. A measurement interval was set to 15 seconds. Based on the resultant luminescence spectrum of polarized luminescence, a luminescence intensity in the wavelength range of from 600 nm to 630 nm was substituted into the equation (1) to obtain an R. The R was measured for 2,400 seconds, and numerical values of time and the R were plotted.

For comparison of the Rs in Examples and Comparative Example, ΔR300-R0 obtained by subtracting an R (R0) immediately after the reaction, that is, immediately after the mixing of the luminescent reagent and the CRP antigen from an R (R300) after 300 seconds from the reaction was determined and compared. Alternatively, ΔR900-R0 obtained by subtracting the R (R0) immediately after the mixing of the CRP antigen from an R (R900) after 900 seconds from the reaction was determined.

Nonspecific agglutination suppression evaluation of the product was performed as described below.

60 μl of a human serum solution diluted 15-fold with a buffer solution was added to the luminescent reagent dispersion (3 mg/mL), and the mixture was kept at a temperature of 37°C for 5 minutes. An absorbance at 527 nm was measured before and after the temperature keeping, and the amount of change in absorbance before and after the temperature keeping was measured 3 times. Table 1 shows the average value of the 3 times. Evaluation was performed as follows: when the amount of change in "absorbance×10,000" value was less than 1,000, it was determined that nonspecific agglutination was suppressed, and when the amount was 1,000 or more, it was determined that nonspecific agglutination occurred.

(Performance Evaluation)

The synthesized luminescent reagent had a particle diameter of about 100 nm, and showed strong red luminescence with excitation light at 340 nm.

According to the results of the nonspecific agglutination suppression evaluation, the change in absorbance was equal to or less than the specific numerical value (the amount of change in "absorbance×10,000" value was 1,000 or less), and hence it was recognized that the particles were capable of suppressing nonspecific adsorption.

The results of Example 1 and Comparative Example 1 are shown in FIG. 2.

FIG. 2 is a graph obtained by plotting the reaction time on the horizontal axis and the R (polarization anisotropic property "r") on the vertical axis. In Example 1, which is plotted with circles in FIG. 2, the R immediately after the reaction is 0.065, and a sharp increase to 0.1 is found at a lapse of 1,000 seconds. Meanwhile, in Comparative Example 1, which is plotted with "×" marks in FIG. 2, the R immediately after the reaction is 0.056, but the R after 1,000 seconds is about 0.064, and hence the difference is small, though the R increases with reaction time. In addition, when the sample of Example 1 was left to stand overnight and then subjected to measurement again, the R was about 0.015. This numerical value is the same as the R of the luminescent reagent in gel, indicating that the polarization anisotropic property is saturated. As apparent from FIG. 2, the R of Example 1 increased to 0.113 after 2,400 seconds, revealing that the reaction was promoted until the R was sufficiently saturated in about 40 minutes of reaction.

The results of Examples 1 to 6 and Comparative Example 1 are shown in Table 1.

In all Examples and Comparative Example, PVP-K30 is present as the hydrophilic polymer at the surface of the luminescent reagent. When the initial values R0 of the polarization anisotropic properties of all Examples and Comparative Example 1 are compared, those of Examples are higher than that of Comparative Example 1, which is 0.05676. This reflects the increase in liquid viscosity due to the addition of the sensitizer. However, the increase in R0 in each of Examples is small as compared to the saturation value of R (about 0.150), and hence falls within a range in which evaluation can be sufficiently performed. In addition, the liquid viscosity of Example 1 was measured to be 2.3 mPa·s. When comparison was performed based on Table 1 in terms of ΔR300-R0 obtained by subtracting the R0 from the value R300 of the polarization anisotropic property after 300 seconds, those of Examples 1,2, and 3, and Comparative Example 1 were 0.0143, 0.0087, 0.0073, 0.007, and 0.00138, respectively, and hence the effects of the sensitizers in Examples were able to be recognized. Further, when comparison was performed based on Table 1 in terms of ΔR900-R0 obtained by subtracting the R0 from the value R900 of the polarization anisotropic property after 900 seconds, those of Examples 1,2, and 3, and Comparative Example 1 were 0.0377, 0.0225, 0.00136, 0.0198, and 0.00594, respectively, and hence it was able to be recognized that the difference between Examples and Comparative Example was further increased. In particular, in the case of Example 1 in which sodium alginate was used as the sensitizer, the numerical value of ΔR300-R0 is 2 or more times as high as the ΔR900-R0 of Comparative Example, showing that the sensitizer can reduce the reaction time in the measurement to one third or less.

In Example 5, it is shown that, when a plurality of hydrophilic polymers are used as the sensitizer, the CRP antigen having a concentration of only 1 pM can be sufficiently detected within 300 seconds (ΔR300-R0=0.00601). In Example 6, it is shown that the sensitizer also has an effect in the case of the TSH antigen-antibody reaction instead of CRP. In addition, it is shown that the use of polyoxazoline as the hydrophilic polymer also has an effect (ΔR300-R0=0.0046). In addition, when an investigation was performed by omitting polyoxazoline from the investigation of Example 6, hardly any change in polarization anisotropic property was able to be observed.

Table 1

| | Example 1 | Example 2 | Example 3 | Example 4 | Example 5 | Example 6 | Comparative Example 1 |
|---|---|---|---|---|---|---|---|
| Polymer present on luminescent particle surface | PVP-K30 | PVP-K30 | PVP-K30 | PVP-K30 | PVP-K30 | PVP-K30 | PVP-K30 |
| Hydrophilic polymer sensitizer added | Sodium alginate | PVP-K90 | PVP-K90 | PVP 1300K | Sodium alginate and PEG | Polyoxazoline | - |
| Amount of hydrophilic polymer sensitizer added | 0.1 w/v% | 0.2 w/v% | 0.4 w/v% | 0.1 w/v% | 0.2 w/v% each | 2.0 w/v% | - |
| Initial value of polarization anisotropic property (R0) | 0.0649 | 0.0620 | 0.0627 | 0.0644 | 0.0789 | 0.0803 | 0.05676 |
| Polarization anisotropic property after 300 seconds (R300) | 0.0792 | 0.0707 | 0.0700 | 0.0714 | 0.0859 | 0.0849 | 0.05814 |

(continued)

| | Example 1 | Example 2 | Example 3 | Example 4 | Example 5 | Example 6 | Comparative Example 1 |
|---|---|---|---|---|---|---|---|
| Polarization anisotropic property after 900 seconds (R900) | 0.1026 | 0.0845 | 0.0763 | 0.0842 | - | - | 0.0627 |
| ΔR300- R0 | 0.0143 | 0.0087 | 0.0073 | 0.007 | 0.007 | 0.0046 | 0.00138 |
| ΔR900- R0 | 0.0377 | 0.0225 | 0.0136 | 0.0198 | - | - | 0.00594 |

Thus, it was shown that the analysis method according to this embodiment enabled measurement of the CRP antigen serving as a target substance with high sensitivity and within a short period of time.

The use of the analysis method according to this embodiment enables measurement of the target substance within a short period of time and with high sensitivity. It is conceived that the use of the analysis method according to this embodiment can realize an apparatus for performing measurement with high sensitivity in an application such as a specimen test in which mass testing is performed in a short period of time.

According to the analysis method according to the present invention, a change in anisotropic property of polarized luminescence can be detected with high sensitivity in correspondence to the agglutination/dispersion behavior of particles, and further, analysis can be performed within a short period of time by virtue of the effect of the sensitizer.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

Provided are a reagent and a measurement method for enabling a specimen test based on polarization anisotropy to be performed with high sensitivity and within a short period of time. Specifically, provided is an analysis method including measuring a value (R) for polarization anisotropy through use of a luminescent reagent that binds with a target substance, the analysis method including: a reaction step including mixing a sample containing the target substance with the luminescent reagent and a sensitizer, and subjecting the mixture to a reaction to obtain a reaction liquid; and a measurement step of measuring the R of the reaction liquid, the luminescent reagent including a luminescent particle substrate and a hydrophilic layer arranged on an outside of the luminescent particle substrate, the sensitizer containing a hydrophilic polymer.

## Claims

1. An analysis method including measuring a value (R) for polarization anisotropy through use of a luminescent reagent that binds with a target substance,

   to thereby determine at least any one of the presence or absence of the target substance and a concentration of the target substance,
   the analysis method comprising:

      a reaction step including mixing a sample containing the target substance with the luminescent reagent and a sensitizer, and subjecting the mixture to a reaction to obtain a reaction liquid; and
      a measurement step of measuring the R of the reaction liquid,
      the luminescent reagent including a luminescent particle substrate and a hydrophilic layer arranged on an outside of the luminescent particle substrate,
      the sensitizer containing a hydrophilic polymer.

2. The analysis method according to claim 1, wherein the hydrophilic polymer is at least one selected from the group consisting of: polyvinylpyrrolidone; sodium alginate; potassium alginate; ammonium alginate; lithium alginate; alginic acid; and polyoxazoline.

3. The analysis method according to claims 1 or 2, wherein the hydrophilic polymer has a molecular weight of 200,000 or more and 2,000,000 or less.

**4.** The analysis method according to any one of claims 1 to 3, wherein the luminescent particle substrate contains a europium complex.

**5.** The analysis method according to any one of claims 1 to 4, wherein the luminescent reagent includes a ligand that binds with the target substance.

**6.** The analysis method according to any one of claims 1 to 5, wherein $R0 \geq 0.001$, where R0 represents the R measured for the luminescent reagent unreacted with the target substance.

**7.** The analysis method according to any one of claims 1 to 6, wherein the R is defined to be "r" in the following equation (1):

$$r = \frac{I_{VV} - G * I_{VH}}{I_{VV} + 2 * G * I_{VH}} \qquad G = \frac{I_{HV}}{I_{HH}} \qquad (1)$$

in the equation (1),

$I_{VV}$ represents a luminescence intensity of a luminescence component having a vibration direction parallel to that of a first polarized light beam at a time of excitation by the first polarized light beam,
$I_{VH}$ represents a luminescence intensity of a luminescence component having a vibration direction orthogonal to that of the first polarized light beam at the time of excitation by the first polarized light beam,
$I_{HV}$ represents a luminescence intensity of a luminescence component having a vibration direction orthogonal to that of a second polarized light beam having a vibration direction orthogonal to that of the first polarized light beam at a time of excitation by the second polarized light beam,
$I_{HH}$ represents a luminescence intensity of a luminescence component having a vibration direction parallel to that of the second polarized light beam having a vibration direction orthogonal to that of the first polarized light beam at the time of excitation by the second polarized light beam, and
G represents a correction value.

**8.** A test kit to be used for analysis involving measuring a value (R) for polarization anisotropy through use of a luminescent reagent that binds with a target substance,

to thereby determine at least any one of the presence or absence of the target substance and a concentration of the target substance,
the test kit comprising:

a first reagent including a luminescent reagent including:

a luminescent particle substrate; and
a hydrophilic layer arranged on an outside of the luminescent particle substrate; and

a second reagent including a sensitizer containing a hydrophilic polymer.

**9.** The test kit according to claim 8, wherein the hydrophilic polymer is at least one selected from the group consisting of: polyvinylpyrrolidone; sodium alginate; potassium alginate; ammonium alginate; lithium alginate; alginic acid; and polyoxazoline.

**10.** The test kit according to claim 8 or 9, wherein the luminescent particle substrate contains a europium complex.

**11.** The test kit according to any one of claims 8 to 10, wherein the luminescent reagent includes a ligand that binds with the target substance.

**12.** A test reagent to be used for analysis involving measuring a value (R) for polarization anisotropy through use of a luminescent reagent that binds with a target substance,

to thereby determine at least any one of the presence or absence of the target substance and a concentration of the target substance,
the test reagent comprising:

a luminescent reagent including:

a luminescent particle substrate; and
a hydrophilic layer arranged on an outside of the luminescent particle substrate; and

a sensitizer containing a hydrophilic polymer.

**13.** The test reagent according to claim 12, wherein the hydrophilic polymer is at least one selected from the group consisting of: polyvinylpyrrolidone; sodium alginate; potassium alginate; ammonium alginate; lithium alginate; alginic acid; and polyoxazoline.

**14.** The test reagent according to claim 12 or 13, wherein the luminescent particle substrate contains a europium complex.

**15.** The test reagent according to any one of claims 12 to 14, wherein the luminescent reagent includes a ligand that binds with the target substance.

FIG. 1

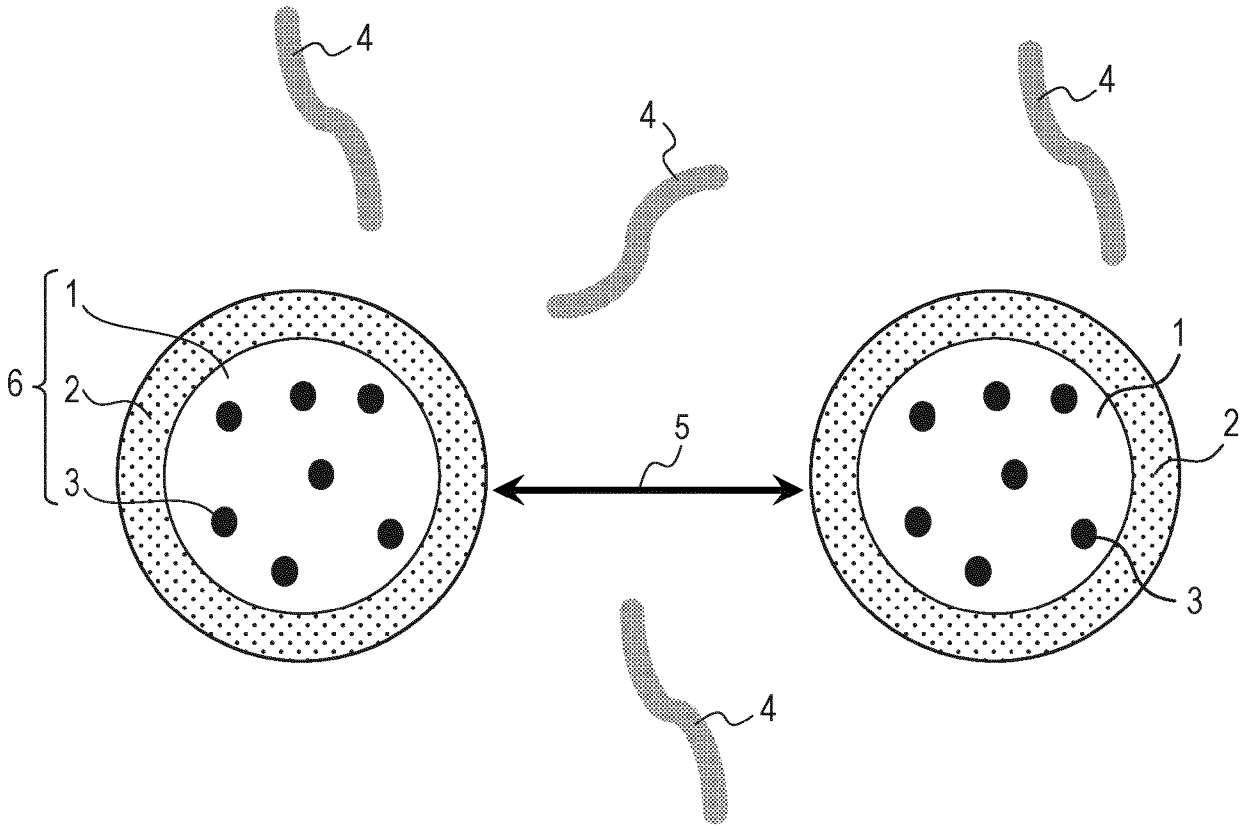

FIG. 2

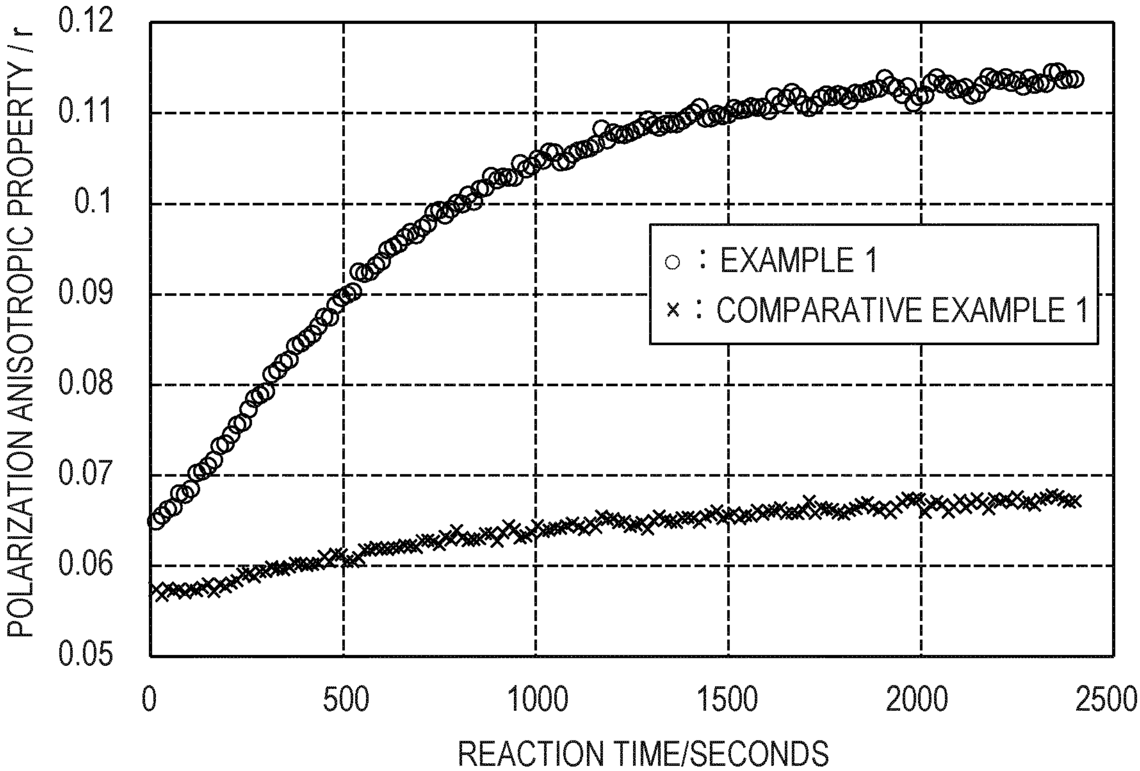
POLARIZATION ANISOTROPIC PROPERTY / r
0.12
0.11
0.1
0.09
0.08
0.07
0.06
0.05
0
500
1000
1500
2000
2500
REACTION TIME/SECONDS
○ : EXAMPLE 1
× : COMPARATIVE EXAMPLE 1

Europäisches Patentamt
European Patent Office
Office européen des brevets

# EUROPEAN SEARCH REPORT

Application Number
EP 23 17 2753

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | KR 2016 0092406 A (KNU INDUSTRY COOPERATION FOUND [KR]) 4 August 2016 (2016-08-04)<br>* paragraphs [0001], [0006], [0007], [0008], [0019], [0032] *<br>----- | 8,9, 11-13,15<br><br>2,5,10, 14 | INV.<br>G01N21/64<br>G01N33/543<br>G01N33/551 |
| Y | US 2010/086488 A1 (HOHEISEL WERNER [DE] ET AL) 8 April 2010 (2010-04-08)<br>* paragraphs [0017], [0018] *<br>----- | 10,14 | |
| X<br><br>Y | GB 2 399 876 A (CATT MICHAEL [GB]) 29 September 2004 (2004-09-29)<br>* page 2, line 14 - page 3, line 15 *<br>* page 4, line 1 - line 14 *<br>* page 2, line 17 *<br>* page 5, line 16 - line 19 *<br>* page 6, line 5 - line 9 *<br>----- | 1,3,4,6, 7<br><br>2,5 | |
| A | DAVID M. JAMESON ET AL: "Fluorescence Polarization/Anisotropy in Diagnostics and Imaging",<br>CHEMICAL REVIEWS,<br>vol. 110, no. 5, 12 May 2010 (2010-05-12), pages 2685-2708, XP055243358,<br>US<br>ISSN: 0009-2665, DOI: 10.1021/cr900267p<br>* the whole document *<br>----- | 1-15 | TECHNICAL FIELDS SEARCHED (IPC)<br><br>G01N |

The present search report has been drawn up for all claims

1

| Place of search | Date of completion of the search | Examiner |
|---|---|---|
| Munich | 27 September 2023 | Brauer, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 17 2753

This annex lists the patent family members relating to the patent documents cited in the above-mentioned European search report. The members are as contained in the European Patent Office EDP file on The European Patent Office is in no way liable for these particulars which are merely given for the purpose of information.

27-09-2023

| Patent document cited in search report | Publication date | Patent family member(s) | Publication date |
|---|---|---|---|
| KR 20160092406 A | 04-08-2016 | NONE | |
| US 2010086488 A1 | 08-04-2010 | AU 2006257017 A1 | 14-12-2006 |
| | | CA 2611171 A1 | 14-12-2006 |
| | | CN 101238196 A | 06-08-2008 |
| | | DE 102005026485 A1 | 14-12-2006 |
| | | EP 1893720 A1 | 05-03-2008 |
| | | JP 2008545980 A | 18-12-2008 |
| | | US 2010086488 A1 | 08-04-2010 |
| | | WO 2006131226 A1 | 14-12-2006 |
| GB 2399876 A | 29-09-2004 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H0352575 B
- JP 2893772 B